# FASCICULE DE BREVET EUROPEEN

(11) **EP 2 396 088 B1**
(45) Date de publication et mention de la délivrance du brevet: **19.12.2018**
(21) Numéro de dépôt: 10707097.1
(22) Date de dépôt: 12.02.2010
(51) Int. Cl.: A61Q 19/10, A61K 8/02, D04H 1/02, D04H 1/46, A61Q 19/00

(54) **ARTICLE POUR LE NETTOYAGE DE LA PEAU**
ARTIKEL ZUR HAUTREINIGUNG
ITEM FOR CLEANING THE SKIN

(30) Priorité: 13.02.2009 FR 0950954
(43) Date de publication de la demande: 21.12.2011
(73) Titulaire: Essity Operations France, 93400 Saint-Ouen (FR)
(72) Inventeur: CLERMONT, Anne-Gaëlle, 68180 Horbourgh Whir (FR); GREGOIRE, Philippe, 27700 Les Andelys (FR); LOUIS DIT PICARD, Bernard, 27370 Le Bosc du Theil (FR); BRET, Bruno, F-68920 Wintzenheim (FR); DA SILVA, Alexandra, F-68600 Neuf Brisach (FR)
(74) Mandataire: Essity Hygiene and Health AB
(86) Numéro de dépôt international: PCT/FR2010/000119
(87) Numéro de publication internationale: WO 2010/092261

(56) Documents cités:
- EP-A- 1 454 577
- WO-A-2005/009191
- FR-A- 2 882 068
- GB-A- 1 285 327
- "Raw cotton primary treatment - vacuum impregnation with surfactant added to the solution improves fibre strength", WPI / THOMSON,, vol. 1976, no. 42, 5 avril 1976 (1976-04-05), XP002550284,

## Description

La présente invention porte sur un procédé de fabrication d'un article pour le nettoyage de la peau, jetable après usage, et sur un article de nettoyage de la peau obtenu par ce procédé. L'article comprend un tampon réalisé en un matériau fibreux formant substrat et une lotion présentant un toucher sensiblement sec sur le substrat.

Dans le domaine cosmétique ou pour les soins du bébé, on utilise généralement, pour nettoyer la peau, un tampon fibreux à base de coton mélangé éventuellement à d'autres fibres synthétiques ou artificielles, sur lequel on dépose une lotion ou émulsion, appropriée, nettoyante ou démaquillante selon le cas.

Il existe maintenant dans le commerce des articles préimprégnés de lotion ou émulsion que l'on peut utiliser directement sans avoir à rapporter de produit nettoyant ou démaquillant supplémentaire au moment de son utilisation.

La présente invention concerne ce type d'articles dans lesquels la lotion présente un aspect et un toucher sensiblement secs sur le substrat, c'est-à-dire que l'article contient globalement une faible quantité d'eau, inférieure à 25%. Il n'est pas utilisé sous cette forme. Pour utiliser l'article, on active la lotion en l'humectant avec de l'eau. La lotion contient des agents par lesquels il se forme une mousse nettoyante lorsque l'article subit une action mécanique, telle qu'une déformation ou un frottement. On applique ensuite le tampon imprégné de mousse humide sur les zones de la peau que l'on souhaite nettoyer.

Les tampons, formant le substrat, que l'on désigne aussi formats sont disponibles sous de nombreuses formes. L'invention vise les tampons obtenus à partir d'une nappe de matière fibreuse en coton seul ou en mélange avec d'autres fibres et dans laquelle ils sont découpés. Généralement, les fibres de la nappe sont liées de manière à former un non tissé à la résistance mécanique plus ou moins grande selon les besoins en tenue de l'article. La forme peut être circulaire, ovale, polygonale ou différente encore, pour des tailles allant de 25 à plus de 100 cm². Les grammages sont compris généralement entre 100 et 300 g/m².

Un tampon fibreux destiné à une telle application est choisi de manière à présenter plusieurs caractéristiques. Il est suffisamment épais pour bien tenir en main lorsqu'il est mouillé. Il absorbe et essuie les impuretés. Il conserve son intégrité, pendant l'usage, au frottement. Il ne peluche pas et ne laisse pas des fibres sur la peau.

On observe que, par rapport à une simple nappe de coton cardée dont ils étaient constitués à l'origine, les propriétés mécaniques des tampons ont été améliorées ces dernières années par la mise en oeuvre de l'une ou l'autre des deux techniques suivantes :
Incorporation dans la masse de fibres d'un liant fusible (sous forme de fibres ou de poudre), avec chauffage par air chaud ou calandrage à chaud ; le liant agglomère les fibres de coton lors de la fusion suivie de son refroidissement et permet d'augmenter la résistance des tampons dans les 3 dimensions. Cette technique n'est cependant pas applicable pour des produits que l'on veut être composés de fibres cellulosiques seulement.

Traitement de la nappe de fibres par des moyens mécaniques, de préférence au moyen de jets d'eau selon un procédé d'hydroliage, qui emmêlent les fibres en épaisseur et en surface.

L'invention concerne un substrat fibreux obtenu, de préférence, selon cette seconde technique.

Le procédé d'hydroliage permet de réduire la propension à la formation de bouloches en surface et d'augmenter la résistance en traction de la nappe. Ce procédé, purement mécanique, permet de fabriquer des nappes de composition allant jusqu'à 100 % de fibres de coton.

Le problème auquel on doit faire face avec un substrat fibreux dont les fibres sont liées mécaniquement est d'éviter une réduction importante de la résistance mécanique du tampon après qu'il a été humidifié et sa désagrégation sous l'effet des contraintes d'étirement auxquelles il est soumis en usage. En effet une composition nettoyante, par une partie des agents qu'elle comprend, implique a priori une plus grande aptitude au glissement entre elles des fibres mouillées.

La perte de résistance n'est pas souhaitée car on veut éviter la désagrégation et la destruction du tampon lorsque l'utilisateur frotte la peau ou toute autre surface.

La demanderesse s'est fixé comme objectif la réalisation d'un article de nettoyage de la peau avec une composition nettoyante que l'on active par mouillage avec de l'eau suivi d'une action mécanique, et incorporée à un substrat fibreux dont les fibres sont liées mécaniquement, qui, lorsqu'il est mouillé, conserve ses propriétés de résistance avant mouillage ou ne les voit pas réduites de façon sensible ou encore les voit améliorées.

La demanderesse s'est également fixé comme objectif un article qui présente une résistance de surface à l'état humide, après mouillage, suffisante pour limiter la formation de bouloches au frottement.

On parvient conformément à l'invention aux objectifs visés avec un procédé de fabrication d'un article de nettoyage de la peau comprenant un substrat fibreux et une composition nettoyante que l'on active par mouillage avec de l'eau suivi d'une action mécanique de manière à obtenir une mousse nettoyante, le procédé comprenant :
- une étape de dépôt sur la surface d'un substrat fibreux sec, qui est un tampon à base de fibres de coton liées par jets d'eau, de grammage compris entre 100 et 300 g/m², d'une lotion aqueuse comprenant au moins un tensioactif, et un humectant tel que la glycérine, et contenant de 25 à 45 % d'eau, de préférence de 30 à 40 % d'eau,
- la quantité d'eau sur le substrat étant inférieure à 25 %, en poids de l'article, et l'article comprenant de 0,1 à 1,2 g de matière active de ladite composition nettoyante par gramme de substrat.

Les matières actives de la composition sont tous les éléments à l'exception de l'eau.

Le substrat présente un grammage compris entre 150 et 250 g/m², plus particulièrement compris entre 160 et 200 g/m².

Conformément à une caractéristique, l'article comprend de préférence de 0,2 à 0,72 gramme de matière active de ladite composition par gramme de substrat. Ladite composition comprend de 15 à 35% dudit tensioactif ou du mélange de tensioactifs exprimé en pourcentage de matière active de la composition et de 55 à 75% dudit humectant exprimé en pourcentage de matière active de la composition.

Conformément à une autre caractéristique, la résistance sens marche de l'article entre son état avant mouillage et son état humide, après mouillage, subit une augmentation d'au moins 10 %.

Conformément à une autre caractéristique, la composition comprend un gélifiant, en une quantité inférieure à 0,2 %. La quantité en matière active du gélifiant est de préférence inférieure à 0,12%.

Avantageusement, la composition comprend au moins un additif tel qu'un agent de conservation, un colorant, un parfum, un régulateur d'acidité, un agent de traitement pour la peau, etc...

Selon un mode de réalisation particulier, le substrat est constitué à 100% de fibres de coton. Le substrat peut cependant contenir un certain pourcentage, de 5 à 30% et plus particulièrement de 15 à 20% de fibres naturelles autres, de fibres synthétiques ou artificielles en substitution des fibres de coton.

Le substrat peut être obtenu à partir d'une nappe de fibres telles que de coton blanchi, formée par voie pneumatique, par cardage ou par une combinaison de ces techniques.

Les couches sont par exemple des voiles de carde. Dans ce dernier cas, un mode de réalisation consiste à replier sur lui-même, au moyen d'un étaleur nappeur, un voile de carde de grammage compris de préférence entre 30 et 60 g/m² selon un angle de nappage compris entre 0 et 90°. On superpose ainsi plusieurs couches jusqu'à obtenir le grammage souhaité.

Le substrat peut aussi être formé d'une pluralité de couches de nature différente. Par exemple le substrat peut être formé à partir d'une nappe obtenue par un dépôt de fibres par voie pneumatique entre deux ou plusieurs voiles de carde.

Selon un autre mode de réalisation, le substrat comprend sur une face un moyen formant exfoliant pour la peau. Avantageusement, il s'agit d'un tampon fabriqué selon la technique divulguée dans la demande de brevet WO200501699 qui porte sur l'incorporation, immédiatement sous la surface du tampon, d'éléments gommants. Ainsi le substrat, conformément à un mode de réalisation, comprend de 5 à 50 g/m² d'éléments gommants. Ce sont des éléments organiques naturels tels que les akènes de fraise, les noyaux d'abricot, la silice organique de bambou ou la cellulose de courge, minéraux tels que les billes de silice, artificiels tels que des sphères de cellulose et de méthylcellulose ou bien encore synthétiques tels que les polymères polyéthylène, nylon, polypropylène ou EVA.

Selon un autre mode de réalisation de nappe avec moyen exfoliant, on mélange aux fibres de coton des fibres de plus gros diamètre et assez rigides. On carde ce mélange pour former un voile qui constitue le voile de surface de la nappe à obtenir. Les fibres rigides sont choisies parmi les fibres de chanvre, de lin, de sisal ou de yuka. Selon l'efficacité de l'exfoliant et la nature de la fibre, on incorpore dans le mélange de 25 à 75% de ces fibres plus rigides dans le voile de surface.

Selon ce procédé on obtient après application de la lotion un article au toucher sensiblement sec qu'il n'est pas nécessaire de sécher pour le conditionner.

Ce procédé permet une application de la lotion dans la masse ou seulement en surface, mais nécessite une étape de séchage.

D'autres caractéristiques et avantages de l'invention apparaîtront plus en détail dans la description qui suit et dans laquelle il est fait référence aux figures :
- la figure 1 est une photographie prise au microscope optique, avec un grossissement de 16 fois, d'une face externe d'un échantillon témoin N1 ayant subi le test de formation de bouloches,
- la figure 2 est une photographie prise au microscope optique avec un grossissement de 16 fois, d'une face externe d'un échantillon N2 conforme à l'invention, ayant subi le test de formation de bouloches,
- la figure 3 est une photographie prise au microscope optique, avec un grossissement de 16 fois, d'une face externe d'un échantillon N3 conforme à l'invention, ayant subi le test de formation de bouloches.

Le substrat est un tampon hydrolié présentant les caractéristiques suivantes :
- Fibres: 100% coton blanchi ;
- Grammage : 180 grammes par m² ;
- Hydroliage : les deux faces ont été soumises à un traitement avec une pluralité de jets d'eau, espacés de 2,5 mm et apport d'une énergie de
   5 à 7x10⁻³ kWh/m² sur une face, espacés de 0,6 mm et apport d'une énergie de 2,08x10⁻³ kWh/m² sur l'autre face.

Plus généralement, dans le cas d'une nappe de 160 à 200 g/m², un mode d'hydroliage comprend le traitement d'une face avec un apport d'énergie entre 4 et 8x10-3kWh/m² et 1 et 3x10-3kWh/m² sur l'autre face.

On rapporte ci-après un mode de fabrication non limitatif d'une nappe fibreuse pour la réalisation d'un tampon formant le substrat selon l'invention.

On réalise une nappe de coton stratifiée selon la technique de fabrication de nappe mentionnée dans le brevet EP 0 681 621 B1.

Cette technique consiste à produire successivement et à superposer 3 couches de coton écru :
- une première couche produite par une carde, par exemple de type pêle-mêle,
- une seconde couche produite par nappage pneumatique à l'aide d'une machine de type Rando, les fibres de la couche étant orientées de manière oblique par rapport aux plans inférieur et supérieur horizontaux de la nappe, et enfin
- une troisième couche produite par une carde et similaire à la première.

Cette nappe composée de trois couches superposées est ensuite traitée chimiquement afin de la rendre hydrophile et blanche. Selon la technique décrite dans ce brevet, la nappe ainsi formée est ensuite entraînée par un tapis support sans fin, perméable aux liquides, vers les différents postes de traitement en ligne continu.

La nappe est imprégnée par déversement par gravité d'une solution de débouillissage contenant de la soude, sur la nappe, sous la forme d'une lame liquide transversale à la direction de déplacement de celle-ci. On crée, au moyen d'une fente d'aspiration disposée sous la toile, une dépression suffisante pour permettre à au moins une partie de la solution de traverser la nappe. On contrôle en même temps la quantité de liqueur apportée à la nappe en réglant le vide créé au niveau de la fente aspirante. La nappe est introduite dans un vaporiseur chauffé à une température voisine de 100°C dans lequel elle séjourne, tout en restant continue, pendant un temps donné notamment en fonction du débit matière.

La nappe est ensuite rincée. Le jus de débouillissage est extrait au moyen d'une deuxième lame liquide et d'une fente à vide associée à un vide moyen. La nappe débouillie et hydrophile est imprégnée d'une solution de blanchiment contenant de l'eau oxygénée, de la même manière que pour le traitement de débouillissage. La nappe est ensuite introduite dans un vaporiseur chauffé à une température d'environ 100°C pour que le blanchiment soit effectif. La nappe est rincée ensuite au moyen d'une succession de lames liquides associées à des fentes aspirantes.

Ce traitement de la nappe confère une adhérence entre les couches la constituant et une très bonne cohésion à l'ensemble. Cette technique permet de fabriquer des nappes de grammage compris entre 80 et 600 g/m².

On améliore la cohésion de cette dernière par un traitement de rinçage selon la technique décrite dans le brevet EP 0 805 888 B1 qui consiste à combiner le rinçage de la nappe avec l'hydroliage par de fins jets d'eau à haute pression, ce qui confère à la nappe, un état de surface lié, sans extrémité libre de fibres et de bonnes résistances mécaniques.

Les jets sont produits par des injecteurs tels qu'utilisés dans la technologie de liage hydrodynamique des nontissés. Chaque injecteur comprend, par exemple, une chambre de forme allongée, fermée sur sa longueur par une plaque perforée, en une ou plusieurs rangées, d'un grand nombre de trous de faible diamètre, de l'ordre de 80 à 200 µm. La chambre est alimentée en liquide sous pression qui s'échappe par les orifices sous la forme de jets fins parallèles de diamètre correspondant.

Le niveau d'énergie à fournir dépend de l'épaisseur de la nappe et de son grammage. La consolidation de la nappe permet sa transformation en tampon absorbant ou tampon à démaquiller par une simple découpe et un conditionnement.

Ce traitement de rinçage appliqué à la nappe stratifiée, à trois couches, décrite ci-dessus présente encore l'avantage de ne pas trop réduire l'épaisseur de la nappe tout renforçant les couches superficielles.

Il est possible de faire subir un traitement de rinçage différencié sur les deux faces. Des traitements différenciés sont décrits dans le brevet EP 1 106 723 B1 ou encore la demande de brevet divisionnaire EP 1 167 605 A1, incorporés ici en référence.

Un exemple de traitement comprend le liage d'une première face par une pluralité de jets d'eau espacés de 2,5 mm, en apportant une énergie de 5 à 7x10⁻³ kWh/m². Le liage de la deuxième face est réalisé au moyen d'une pluralité de jets d'eau espacés de 0,6 mm, apportant une énergie de 2,08x10⁻³ kWh/m². Après liage la nappe est séchée et découpée en tampons au format souhaité par des moyens de découpe appropriés.

### Composition de la lotion

Conformément à l'invention, la lotion comprend au moins les composants suivants :
- Un agent humectant tel que la glycérine,
- Un tensioactif tel qu'un tensioactif non ionique, anionique ou amphotère susceptible de former la mousse.

Les additifs optionnels sont les suivants :
Agent de conservation tel que ceux autorisés en cosmétique et formant la liste publiée au journal officiel en application de la directive n°76/768/CEE,
Parfum,
Colorant,
Agent de traitement de la peau,
Gélifiant,
Régulateur d'acidité.

Comme agent humectant, c'est à dire un agent hygroscopique permettant de retenir l'eau et donc d'améliorer l'hydratation de la peau, outre la glycérine, on peut utiliser un autre polyol comme le propylène glycol, le butylène glycol, le sorbitol, le pentylène glycol ou l'hexylène glycol.

L'agent tensioactif non ionique est de préférence choisi parmi les alkyl poly glucosides tel que par exemple le Decyl Glucoside.

L'agent tensioactif anionique est de préférence un ou plusieurs des agents présentant l'un des trois groupes fonctionnels suivants :
- sulfates tels que les alkyl sulfates, les alkyl éther sulfates dont le Sodium Laureth Sulfate, les disodium sulfosuccinates,
- sulfonates tels que les alkyl taurates, les alkyl iséthionates, les alkyl aryl sulfonates ou les oléfines sulfonates ;
- carboxylates tels que les sarcosinates ou les acyl glutamates.

L'agent tensioactif amphotère est de préférence un ou plusieurs des agents suivants, selon la classification d'après « Handbook of Surfactants » M.R. Porter, 2ème édition, Blackie A&P ;

Les dérivés d'amino propionate tel que alkyl aminopropionates, alkyl ampho polycarboxy propionates, ampho propionates, ampho carboxy propionates, alkyl imino dipropionates, amino alkanoates, beta-N-alkylalanines, alkyl amino propionates, alkyl iminodipropionates, imino dialkanoate propionates.

Les dérivés de bétaïne, par exemple Cocamidopropyl Betain, les alkyl telqu'alkyl bétaïnes, alkyl bisbétaïnes, alkyl diméthyl bétaïnes , alkyl amido bétaïnes, alkyl amido propyl bétaïnes, alkyl amido propyl dimethyl bétaïne, alkyl amido propyl dimethyl sulphobétaïnes, alkyl amido propyl hydroxy sultaines ; les sulpho amido bétaïnes ; les sulpho bétaïnes.

Les glycinates tels que les alkyl glycinates, alkyl amino acides carboxyliques, alkyl amphomonoacétates, alkyl amphodiacétates, alkyl carboxy glycinates, alkyl ampho polycarboxy glycinates, alkyl imino diglycinates, alkyl polyaminocarboxylates, amino alkanoates, ampho glycinates, ampho carboxy glycinates, carboxy glycinates, alpha-N-alkyl amino acides acétiques.

### ESSAIS

On a précédé à des essais visant à vérifier les propriétés de résistance mécanique d'une part et de résistance au frottement de surface d'autre part.

Trois articles ont été testés.

Un échantillon de coton témoin N1 tel que défini dans l'exemple de réalisation ci-dessus à savoir : 100% coton blanchi, 180 g/m², les deux faces de la nappe ont été soumises à un traitement avec une pluralité de jets d'eau, espacés de 2,5 mm et apport d'une énergie de 6,7x10⁻³ kWh/m² sur une face, espacés de 0,6 mm et apport d'une énergie de 2,08x10⁻³ kWh/m² sur l'autre face.

Un tampon imprégné d'une lotion dont la composition est la suivante :

| | |
|---|---|
| Glycerin | : 43,27 |
| Eau | : 35,94 |
| Tensioactif anionique - Laureth Sodium Sulfate | : 11,52 |
| Tensioactif amphotère : Cocamido Propyl Betain | : 1,92 |
| Parfum | : 1,8 |
| TA non ionique-Decyl Glucoside | : 2,26 |
| Conservateur - | : 1,30 |
| Potassium Sorbate | : 0,29 |
| Citric Acid | : 0,18 |
| Extrait d'algue | : 1,50 |
| Colorant | : 0,02 |
| Total | : 100 |

Les matières actives de la composition sont tous les éléments à l'exception de l'eau.

Un échantillon N2 est préparé par le dépôt de la lotion ci-dessus sur le substrat à un taux de 0,56 gramme de lotion par gramme de substrat. La lotion étant relativement visqueuse, le dépôt est effectué au moyen, d'une buse à lèvre. L'article obtenu présente un taux de siccité suffisant pour ne pas avoir à le sécher.

Un échantillon N3 est préparé avec une lotion de même composition que ci-dessus, diluée 4 fois dans l'eau. Le dépôt est effectué par foulardage, suivi d'un séchage. Le taux d'imprégnation de matières actives final est le même que pour N2.

Test de mesure de la résistance à la traction

On a appliqué le test EDANA référencé 20.2-89 qui permet de qualifier le comportement de textiles nontissés soumis à des contraintes de traction.

Les valeurs de résistance à la traction ont été mesurées sur des échantillons aux dimensions spécifiées dans le test sur lesquels on a appliqué une force de traction longitudinale à une vitesse d'accroissement constante.

On a procédé aux essais sur des échantillons avant mouillage d'une part et sur des échantillons qui ont été humidifiés, après mouillage, d'autre part. Avant mouillage, pour N1 son humidité résiduelle est inférieure à 8%, cas du coton par exemple, pour N2 et N3 son humidité résiduelle est inférieure à 25% en poids de l'article.

Pour l'humidification, l'échantillon a été plié en deux dans son milieu, la pliure étant perpendiculaire à la longueur de l'échantillon. La zone de la pliure sur un centimètre est trempée une seconde dans de l'eau distillée, sans égouttage. L'échantillon est déplié pour la mesure.

On a procédé à 50 essais pour chacune des trois nappes, N1, N2, N3, respectivement avant mouillage et après mouillage. Les valeurs moyennes, maximales et minimales des valeurs de résistance sur des échantillons découpés dans le sens marche d'une part avant mouillage (RSMS) et d'autre part après mouillage (RSMH) en Newton sont résumées dans le tableau ci-dessous :

| | **N1** | | **N2** | | **N3** | |
|---|---|---|---|---|---|---|
| | **RSMS** | **RSMH** | **RSMS** | **RSMH** | **RSMS** | **RSMH** |
| **Moyenne** | **27,82** | **20,49** | **14,76** | **18,57** | **11,46** | **24,61** |
| **Maxi** | **34,61** | **27,42** | **22,46** | **25,99** | **16,89** | **30,30** |
| **Mini** | **23,59** | **15,29** | **8,67** | **12,06** | **8,08** | **20,31** |
| **Ecart-type** | **2,58** | **2,91** | **3,44** | **2,32** | **1,92** | **2,54** |

La comparaison des valeurs obtenues avant mouillage et après mouillage donne les variations suivantes pour chaque échantillon par rapport à lui-même.
N1 : -26%
N2 : +26%
N3 : +115%

Les résistances avant mouillage montrent que l'imprégnation fait perdre de la résistance, la valeur de la résistance de la nappe imprégnée dans la masse étant plus faible que celle de la nappe imprégnée en surface.

De façon surprenante, après humidification, on améliore les caractéristiques de résistance du tampon contenant la composition nettoyante, soit en surface soit imprégné dans l'épaisseur, alors que l'on s'attendait à une chute supplémentaire. On récupère une certaine résistance qui va au-delà de celle du tampon témoin pour le tampon imprégné dans la masse.

### Test de formation de bouloches.

Selon ce test, les nappes sont humidifiées par foulardage (la pression de foulardage est réglée au minimum 0 et la vitesse de défilement est de 0,7m/min). Le taux d'emport est d'environ 150%. Elles sont ensuite soumises à un frottement conformément à la méthode MARTINDALE version pilling, (NF EN ISO 12945-2) :
sans charge (la masse du porte éprouvette ensemble avec la tige est de 155g),
sans feutre sous l'échantillon,
en remplaçant la toile de laine par le tissu utilisé pour les tests de solidité des teintures au frottement selon la norme NF EN ISO 105x12, et
en procédant à l'examen des éprouvettes après 5 et 10 cycles.

Les bouloches formées sur le nontissé sont comptées et mesurées par analyse optique.

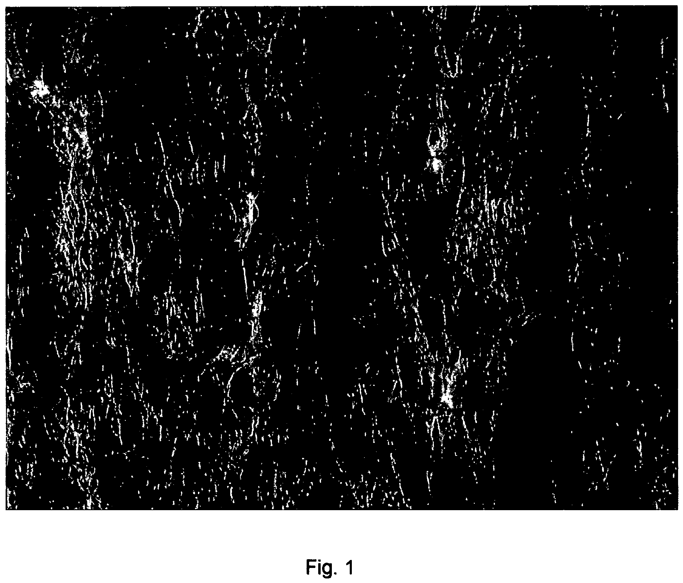

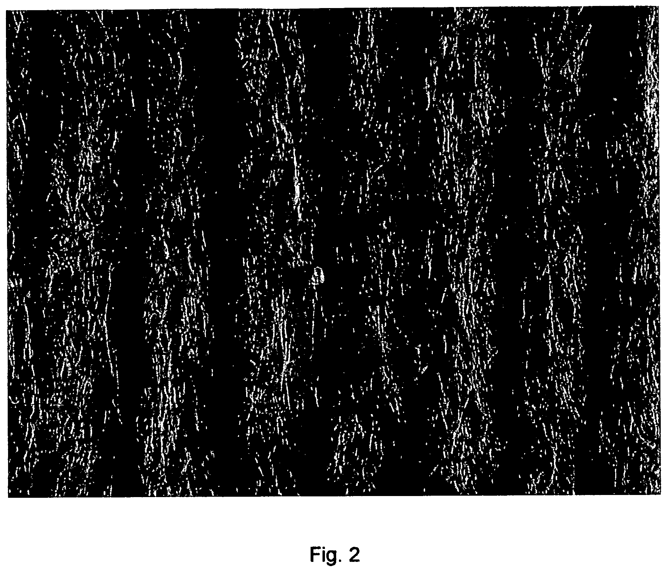

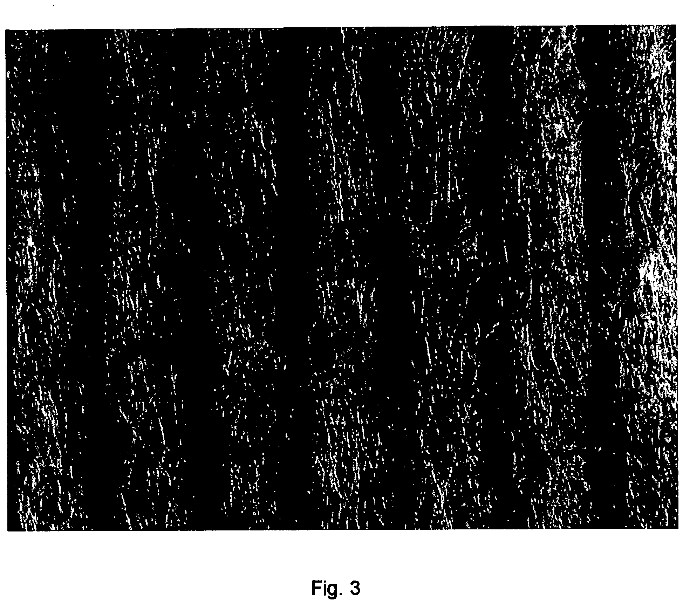

Sur une surface totale de 2084 mm², on mesure le nombre de bouloches et leur surface cumulée. On a photographié les échantillons ayant subi le test, le résultat apparaît sur les figures 1 à 3, pour respectivement, les échantillons N1 à N3.

Test avec 5 cycles
N1 : 116 bouloches ayant une surface totale de 21 mm² ;
N2 : 41 bouloches ayant une surface totale de 9 mm² ;
N3 : 43 bouloches ayant une surface totale de 6 mm² ;

Test avec 10 cycles
N1 : 178 bouloches ayant une surface totale de 34 mm² ;
N2 : 60 bouloches ayant une surface totale de 8 mm² ;
N3 : 69 bouloches ayant une surface totale de 9 mm²

Ces résultats mettent en avant le produit N2 qui présente non seulement un gain de résistance lors de l'humidification mais aussi une cohésion de surface. Moins de bouloches sont formées ; et elles sont de plus petite taille.

## Revendications

1. Procédé de fabrication d'un article de nettoyage de la peau comprenant un substrat fibreux et une composition nettoyante que l'on active par mouillage avec de l'eau suivi d'une action mécanique de manière à obtenir une mousse nettoyante, le procédé comprenant :
- une étape de dépôt sur la surface d'un substrat fibreux sec, qui est un tampon à base de fibres de coton liées par jets d'eau, de grammage compris entre 100 et 300 g/m², d'une lotion aqueuse comprenant au moins un tensioactif, et un humectant tel que la glycérine, et contenant de 25 à 45 % d'eau, de préférence de 30 à 40 % d'eau,
- la quantité d'eau sur le substrat étant inférieure à 25 %, en poids de l'article, et l'article comprenant de 0,1 à 1,2 g de matière active de ladite composition nettoyante par gramme de substrat.

2. Procédé selon la revendication 1, dans lequel le grammage du substrat est compris entre 150 et 250 g/m², plus particulièrement de 160 à 200 g/m².

3. Procédé selon la revendication 1 ou 2, dans lequel l'article obtenu comprend de 0,2 à 0,72 gramme de matière active par gramme de substrat.

4. Procédé selon l'une quelconque des revendications précédentes, dans lequel la composition nettoyante comprend un mélange de tensioactifs choisis parmi les tensioactifs nonioniques, anioniques et amphotères.

5. Procédé selon l'une quelconque des revendications précédentes dans lequel la composition nettoyante comprend de 15 à 35 % du au moins un tensioactif et de 55 à 75% dudit humectant.

6. Procédé selon l'une quelconque des revendications précédentes, dans lequel la composition nettoyante comprend de plus un gélifiant en une quantité inférieure à 0,2%, de préférence inférieure à 0,12%.

7. Procédé selon l'une quelconque des revendications précédentes, dans lequel la composition nettoyante comprend de plus un additif tel qu'un agent de conservation, un colorant, un parfum ou un régulateur d'acidité.

8. Procédé selon l'une quelconque des revendications précédentes, dans lequel le substrat fibreux comprend sur une face au moins, un moyen formant exfoliant pour la peau.

9. Procédé selon la revendication 8, dans lequel le substrat comprend de 5 à 50 g/m² d'éléments gommants formant matière exfoliante.

10. Article de nettoyage de la peau obtenu par le procédé selon l'une quelconque des revendications précédentes, dont le nombre de bouloches, après frottement selon la méthode martindale version pilling (NF EN ISO 12945-2), est de moitié au moins par rapport au dit substrat, sans lotion.

## Patentansprüche

1. Verfahren zur Herstellung eines Artikels zur Hautreinigung, umfassend ein Fasersubstrat und eine Reinigungszusammensetzung, die durch Befeuchten mit Wasser aktiviert wird, gefolgt von mechanischer Einwirkung, um einen Reinigungsschaum zu erhalten, wobei das Verfahren umfasst:
- einen Schritt zum Auftragen auf die Oberfläche eines trockenen Fasersubstrats, bei dem es sich um ein Pad, bestehend aus wasserverfestigten Baumwollfasern, mit einem Flächengewicht im Bereich zwischen 100 und 300 g/m² handelt, einer wässrigen Lotion, die mindestens ein Tensid und ein Feuchthaltemittel, wie beispielsweise Glycerin, umfasst und 25 bis 45 % Wasser, vorzugsweise 30 bis 40 % Wasser, enthält,
- wobei die Wassermenge auf dem Substrat weniger als 25 Gew.-% des Artikels beträgt und der Artikel 0,1 bis 1,2 g aktives Material der Reinigungszusammensetzung je Gramm Substrat umfasst.

2. Verfahren nach Anspruch 1, wobei das Flächengewicht des Substrats im Bereich zwischen 150 und 250 g/m², insbesondere 160 und 200 g/m² liegt.

3. Verfahren nach Anspruch 1 oder 2, wobei der erhaltene Artikel 0,2 bis 0,72 Gramm aktives Material je Gramm Substrat umfasst.

4. Verfahren nach einem der vorhergehenden Ansprüche, wobei die Reinigungszusammensetzung eine Mischung von Tensiden umfasst, ausgewählt aus nichtionischen, anionischen und amphoteren Tensiden.

5. Verfahren nach einem der vorhergehenden Ansprüche, wobei die Reinigungszusammensetzung 15 bis 35 % des mindestens einen Tensids und 55 bis 75 % des Feuchthaltemittels umfasst.

6. Verfahren nach einem der vorhergehenden Ansprüche, wobei die Reinigungszusammensetzung ferner einen Gelbildner in einer Menge von weniger als 0,2 %, vorzugsweise weniger als 0,12 %, umfasst.

7. Verfahren nach einem der vorhergehenden Ansprüche, wobei die Reinigungszusammensetzung ferner einen Zusatzstoff, wie beispielsweise ein Konservierungsmittel, einen Farbstoff, ein Parfüm oder einen Säureregulator, umfasst.

8. Verfahren nach einem der vorhergehenden Ansprüche, wobei das Fasersubstrat auf einer Seite mindestens ein Mittel umfasst, das ein Peeling für die Haut bildet.

9. Verfahren nach Anspruch 8, wobei das Substrat 5 bis 50 g/m² schälende Elemente die das Peeling-Material bilden, umfasst.

10. Artikel zur Hautreinigung, der durch das Verfahren nach einem der vorhergehenden Ansprüche erhalten wird, wobei die Anzahl an Flusen, nach dem Scheuern gemäß dem Martindale-Verfahren zur Pillneigung (NF EN ISO 12945-2), mindestens halbiert ist, bezogen auf das Substrat ohne Lotion.

## Claims

1. Method for producing an item for cleansing the skin, comprising a fibrous substrate and a cleansing composition that is activated by moistening with water followed by a mechanical action so as to obtain a cleansing foam, the method comprising:
- a step of depositing on the surface of a dry fibrous substrate, which is a pad based on water-jet-entangled cotton fibres with a grammage of between 100 and 300 g/m², an aqueous lotion comprising at least one surfactant, and a humectant such as glycerol, and containing from 25% to 45% of water, preferably from 30% to 40% of water,
- the amount of water on the substrate being less than 25% by weight of the item, and the item comprising from 0.1 to 1.2 g of active material of said cleansing composition per gram of substrate.

2. Method according to Claim 1, in which the grammage of the substrate is between 150 and 250 g/m², more particularly from 160 to 200 g/m².

3. Method according to Claim 1 or 2, in which the item obtained comprises from 0.2 to 0.72 grams of active material per gram of substrate.

4. Method according to any one of the preceding claims, in which the cleansing composition comprises a mixture of surfactants chosen from non-ionic, anionic and amphoteric surfactants.

5. Method according to any one of the preceding claims, in which the cleansing composition comprises from 15% to 35% of at least one surfactant and from 55% to 75% of said humectant.

6. Method according to any one of the preceding claims, in which the cleansing composition also comprises a gelling agent in an amount of less than 0.2%, preferably less than 0.12%.

7. Method according to any one of the preceding claims, in which the cleansing composition also comprises an additive such as a preserving agent, a dye, a fragrance or an acidity regulator.

8. Method according to any one of the preceding claims, in which the fibrous substrate comprises, on at least one face, a means forming a skin exfoliant.

9. Method according to Claim 8, in which the substrate comprises from 5 to 50 g/m² of scrubbing elements forming an exfoliant material.

10. Item for cleansing the skin, obtained by means of the method according to any one of the preceding claims, the number of pills of which, after rubbing according to the Martindale method, pilling version (NF EN ISO 12945-2), is at least halved relative to said substrate, without lotion.
